(19) Europäisches Patentamt
European Patent Office
Office européen des brevets

(11) Veröffentlichungsnummer: **0 139 183**
**A2**

(12) # EUROPÄISCHE PATENTANMELDUNG

(21) Anmeldenummer: **84110330.2**

(22) Anmeldetag: **30.08.84**

(51) Int. Cl.[4]: **A 01 N 43/56**
A 01 N 43/653

(30) Priorität: **07.09.83 DE 3332271**

(43) Veröffentlichungstag der Anmeldung:
**02.05.85 Patentblatt 85/18**

(84) Benannte Vertragsstaaten:
**AT BE CH DE FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Knops, Hans-Joachim, Dr.**
**Köpenicker Strasse 35**
**D-4019 Monheim(DE)**

(72) Erfinder: **Lunkenheimer, Winfried, Dr.**
**Bismarckstrasse 29**
**D-5600 Wuppertal 1(DE)**

(72) Erfinder: **Fedtke, Carl, Dr.**
**Wehrheiderstrasse 5**
**D-5000 Köln-Dellbrück(DE)**

(54) **Herbizide Mittel Enthaltend Photosynthesehemmer-Herbizide in Kombination mit substituierten Azolyl-Derivaten.**

(57) Die neuen synergistischen Wirkstoffkombinationen, bestehend aus (1) einem Photosynthesehemmer-Wirkstoff (Herbizid) und (2) einem substituierten Azolyl-Derivat der allgemeinen Formeln (II), (III) und (IV) (Synergisten),

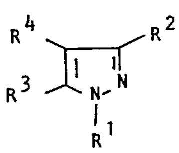

(II)

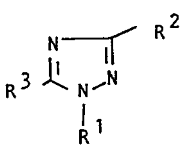

(III)

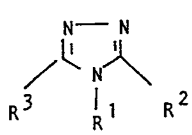

(IV)

in welchen
$R^1$ für Wasserstoff oder Alkoxycarbonylmethyl steht,
$R^2$ für Wasserstoff oder Alkyl steht,
$R^3$ für Wasserstoff, Alkyl oder Alkylthio steht und
$R^4$ für Wasserstoff oder Halogen steht,
weisen eine besonders hohe herbizide Wirksamkeit auf.

Charakteristische Beispiele für die Photosynthesehemmer-Wirkstoffe sind Metribuzin, Methabenzthiazuron, Linuron sowie 4-Amino-6-tert.-butyl-3-ethylthio-1,2,4-triazin-5-on.

EP 0 139 183 A2

Croydon Printing Company Ltd

BAYER AKTIENGESELLSCHAFT 5090 Leverkusen, Bayerwerk
Konzernverwaltung RP
Patentabteilung Bi/AB
III

# Herbizide Mittel enthaltend Photosynthesehemmer-Herbizide in Kombination mit substituierten Azolyl-Derivaten

Die vorliegende Erfindung betrifft neue herbizide synergistische Wirkstoffkombinationen, die aus bekannten Photosynthesehemmer-Herbiziden einerseits und aus bestimmten, weitgehend bekannten substituierten Azolyl-Derivaten andererseits bestehen.
Es ist bereits bekannt geworden, daß bestimmte Herbizide wie z.B. 4-Amino-6-tert.-butyl-3-methylthio- bzw. ethylthio -1,2,4-triazin-5-on; 1-Methoxy-1-methyl-3-(3,4-dichlorphenyl)-harnstoff oder 1,3-Dimethyl-1-(benzo-1,3-thiazol-2-yl)-harnstoff photosynthese-hemmende Eigenschaften besitzen (vergl. z.B. Carl Fedtke, Biochemistry and Physiology of Herbicide Action, Springer-Verlag, 1982). Nachteilig bei diesen herbiziden Verbindungen ist jedoch, daß nicht immer alle vorkommenden Unkräuter und Ungräser voll erfaßt werden oder aber, daß bei entsprechend hohen Aufwandmengen einige Kulturpflanzen teilweise geschädigt werden.

Es wurde gefunden, daß die neuen Wirkstoffkombinationen, bestehend aus

a) einem Photosynthesehemmer-Wirkstoff (Herbizid)

und

b) einem substituierten Azolyl-Derivat der allgemeinen Formeln (II), (III) und (IV) (Synergisten)

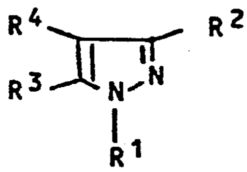

(II)

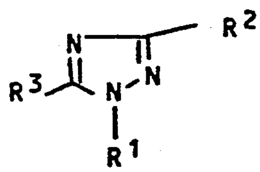

(III)

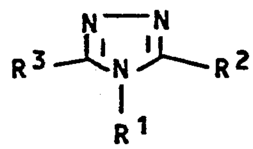

(IV)

in welchen

$R^1$ für Wasserstoff oder Alkoxycarbonylmethyl steht,

$R^2$ für Wasserstoff oder Alkyl steht,

$R^3$ für Wasserstoff, Alkyl oder Alkylthio steht und

$R^4$ für Wasserstoff oder Halogen steht,

eine besonders hohe herbizide Wirksamkeit aufweisen.

Ueberraschenderweise ist die herbizide Wirksamkeit der erfindungsgemäßen Wirkstoffkombinationen wesentlich höher als die Summe der Wirkungen der einzelnen Wirkstoffe. Insbesondere besitzen die weitgehend bekannten Azolyl-Derivate der allgemeinen Formeln (II), (III) und (IV) bei den üblichen Aufwandmengen keine nennenswerte eigene herbizide Wirkung, bewirken aber die Steigerung der herbiziden Wirkung der Photosynthesehemmer-Wirkstoffe. Somit ist der hier gefundene synergistische Effekt völlig unerwartet und überraschend.

Da der synergistische Effekt auch solche Unkräuter betrifft, die durch die verwendeten Photosynthesehemmer-Wirkstoffe bei alleiniger Ausbringung in üblichen Aufwandmengen nur unzureichend geschädigt oder gar nicht erfaßt werden, stellen die erfindungsgemäßen synergistischen Wirkstoffkombinationen eine wertvolle Bereicherung der Technik dar.

Als Photosynthesehemmer-Wirkstoffe für die erfindungsgemäßen Wirkstoffkombinationen seien vorzugsweise die folgenden der allgemeinen Formeln (I-A) bis (I-J) genannt:

(A) Triazinon-Derivate der Formel

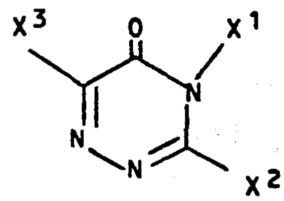

(I-A)

in welcher

$X^1$ für Amino, gegebenenfalls substituiertes Alkylidenamino oder Alkyl mit 1 bis 2 Kohlenstoffatomen steht;

$X^2$ für Alkylthio mit 1 bis 2 Kohlenstoffatomen, Alkyl- und Dialkylamino mit jeweils 1 bis 2 Kohlenstoffatomen in jedem Alkylteil oder Alkyl mit 1 bis 4 Kohlenstoffatomen steht; und

$X^3$ für gegebenenfalls durch Halogen substituiertes tert.-Butyl oder gegebenenfalls substituiertes Phenyl steht;

(B) Triazindion-Derivate der Formel

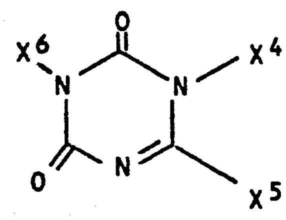

(I-B)

in welcher

$X^4$ für Amino, gegebenenfalls substituiertes Alkylidenamino oder Alkyl mit 1 bis 2 Kohlenstoffatomen steht;

$X^5$ für Alkylthio mit 1 bis 2 Kohlenstoffatomen, Alkyl- und Dialkylamino mit jeweils 1 bis 2 Kohlenstoffatomen in jedem Alkylteil oder Alkyl mit 1 bis 4 Kohlenstoffatomen steht; und

$X^6$ für Alkyl mit 1 bis 6 Kohlenstoffatomen oder gegebenenfalls substituiertes Phenyl steht;

(C) Triazin-Derivate der Formel

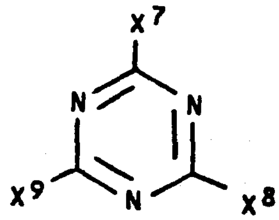

(I-C)

in welcher

$X^7$ für Chlor, Alkoxy oder Alkylthio mit jeweils 1 bis 2 Kohlenstoffatomen steht;

$X^8$ für Alkylamino mit 1 bis 4 Kohlenstoffatomen im Alkylteil steht; und

$X^9$ für gegebenenfalls durch Cyano substituiertes Alkyl mit 1 bis 4 Kohlenstoffatomen steht;

(D) Harnstoff-Derivate der Formel

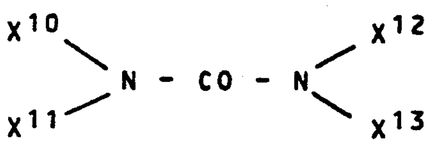

(I-D)

in welcher

$X^{10}$ für gegebenenfalls substituiertes Phenyl, Benzthiazolyl oder gegebenenfalls substituiertes Thiadiazolyl steht;

$X^{11}$ für Wasserstoff oder Methyl steht;

$X^{12}$ für Methyl steht; und

$X^{13}$ für Wasserstoff, Alkyl mit 1 bis 4 Kohlenstoffatomen, Alkoxy mit 1 bis 2 Kohlenstoffatomen oder Alkinyl mit 2 bis 4 Kohlenstoffatomen steht;

(E) Carboxanilid-Derivate der Formel

$$X^{14} - CO - NH - X^{15} \qquad \text{(I-E)}$$

in welcher

$X^{14}$ für Alkyl mit bis zu 6 Kohlenstoffatomen, Alkoxy mit 1 bis 4 Kohlenstoffatomen, Alkenyl mit 2 bis 4 Kohlenstoffatomen oder Cycloalkyl mit 3 bis 6 Kohlenstoffatomen steht; und

$X^{15}$ für gegebenenfalls substituiertes Phenyl steht;

(F) Uracil-Derivate der Formel

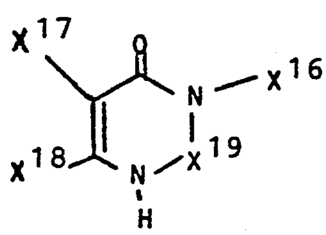

(I-F)

in welcher

$X^{16}$ für Alkyl mit 1 bis 6 Kohlenstoffatomen oder Cycloalkyl mit 5 bis 7 Kohlenstoffatomen steht;

$X^{17}$ für Halogen steht;

$X^{18}$ für Alkyl mit 1 bis 2 Kohlenstoffatomen steht, oder

$X^{17}$ und $X^{18}$ gemeinsam für eine gegebenenfalls substituierte Alkylenkette oder einen gegebenenfalls substituierten anellierten Benzolring stehen; und

$X^{19}$ für die -CO-oder $-SO_2$-Gruppe steht;

(G) Biscarbamat-Derivate der Formel

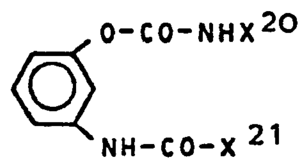

(I-G)

in welcher

$X^{20}$ für Alkyl mit 1 bis 4 Kohlenstoffatomen oder gegebenenfalls substituiertes Phenyl steht; und

$X^{21}$ für Alkoxy mit 1 bis 4 Kohlenstoffatomen oder Dialkylamino mit 1 bis 2 Kohlenstoffatomen in jedem Alkylteil steht;

(H) Pyridazinon-Derivate der Formel

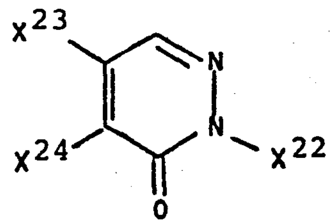

(I-H)

in welcher

$X^{22}$ für gegebenenfalls substituiertes Phenyl steht;

$X^{23}$ für Amino, Alkylamino oder Dialkylamino mit jeweils 1 oder 2 Kohlenstoffatomen in jedem Alkylteil steht; und

$X^{24}$ für Halogen steht;

(J) Hydroxybenzonitril-Derivate der Formel

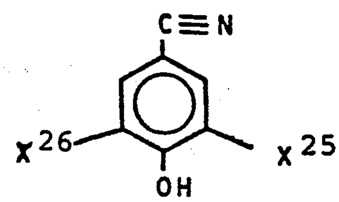

(I-J)

in welcher

$X^{25}$ für Halogen steht; und

$X^{26}$ für Halogen steht.

Besonders bevorzugt sind folgende Photosynthesehemmer-Wirkstoffe der allgemeinen Formeln (I-A) bis (I-J):

(A) Triazinon-Derivate der Formeln

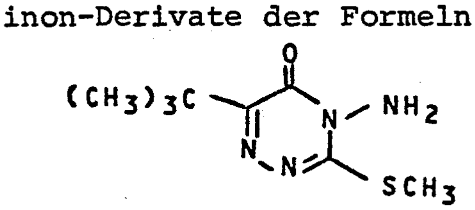

(I-A-1)
(Metribuzin)

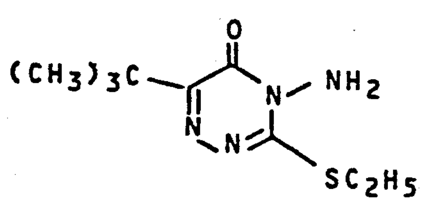

(I-A-2)

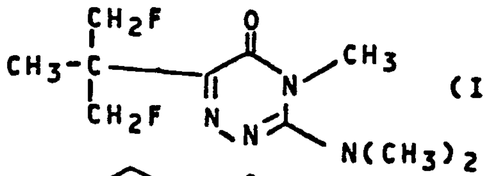

(I-A-3)

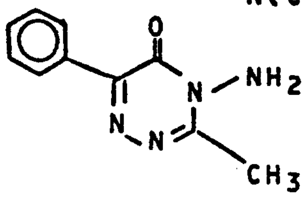

(I-A-4)
(Metamitron)

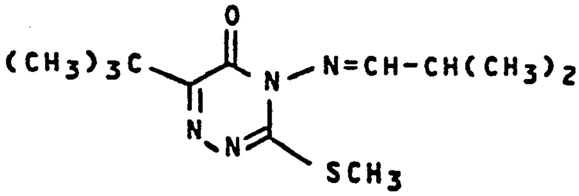

(I-A-5)
(Isomethiozin)

(B) Triazindion-Derivate der Formeln

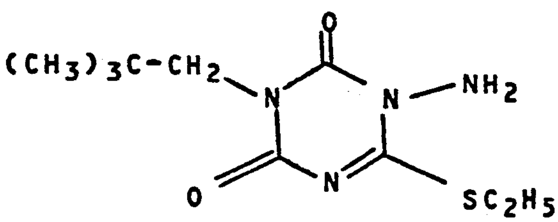

(I-B-1)
(Ametridione)

(C) Triazin-Derivate der Formeln

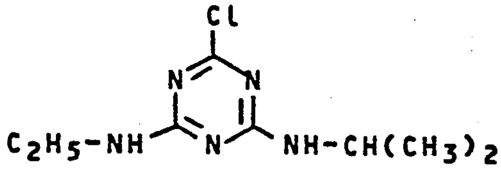

(I-C-1)
(Atrazine)

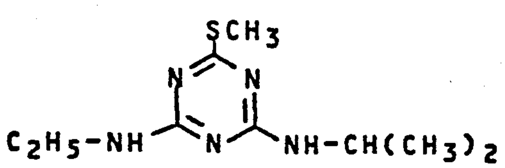

(I-C-2)
(Ametryne)

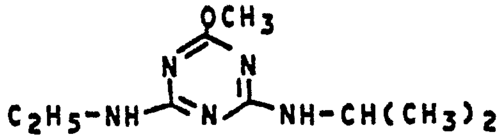

(I-C-3)
(Atraton)

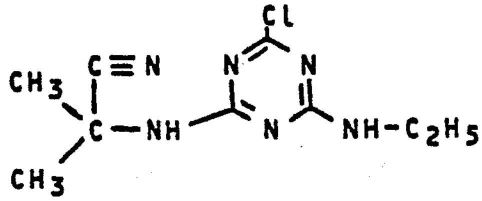

(I-C-4)
(Cyanazine)

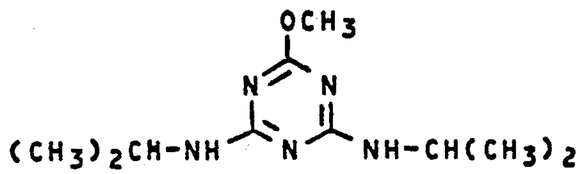

(I-C-5)
(Prometon)

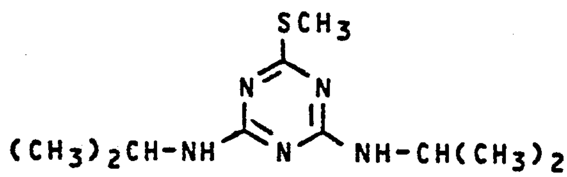

(I-C-6)
(Prometryne)

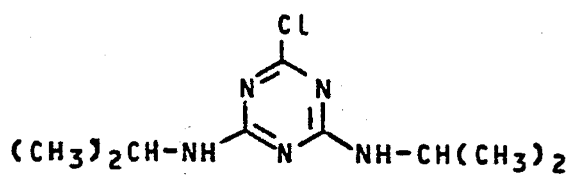

(I-C-7)
(Propazine)

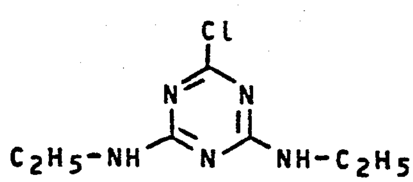

(I-C-8)
(Simazine)

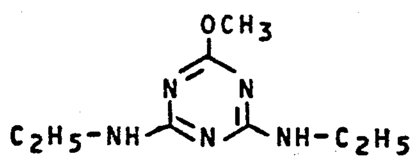

(I-C-9)
(Simeton)

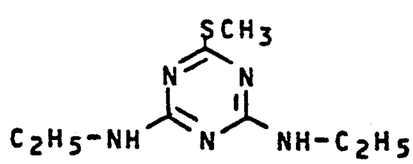

(I-C-10)
(Simetryne)

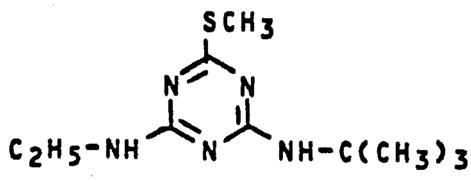

(I-C-11)
(Terbutryne)

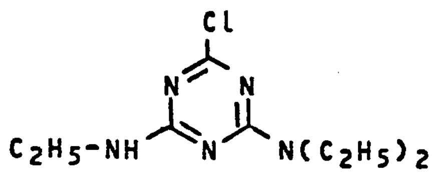

(I-C-12)
(Trietazine)

(D) Harnstoff-Derivate der Formeln

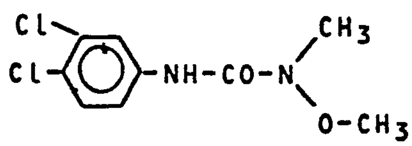

(I-D-1)
(Linuron)

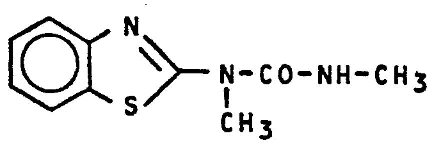

(I-D-2)
(Methabenzthiazuron)

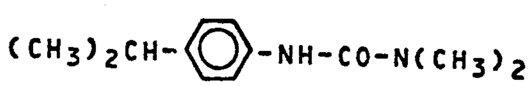

(I-D-3)
(Isoproturon)

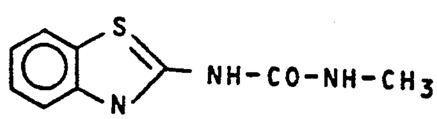

(I-D-4)
(Benzthiazuron)

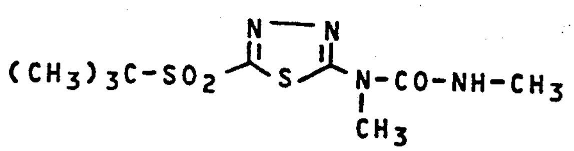

(I-D-5)
(Buthiuron)

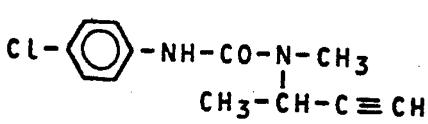

(I-D-6)
(Buturon)

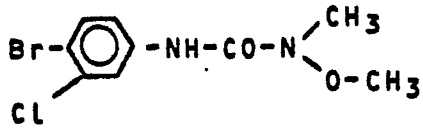

(I-D-7)
(Chlorbromuron)

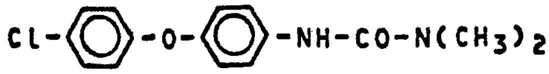

(I-D-8)
(Chloroxuron)

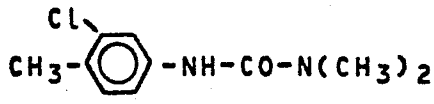

(I-D-9)
(Chlortoluron)

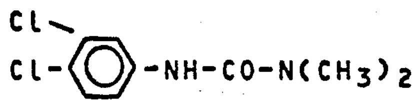

(I-D-10)
(Diuron)

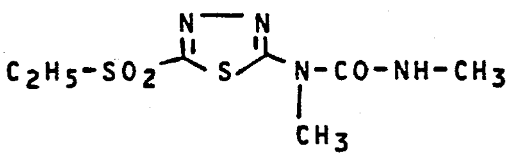

(I-D-11)
(Ethidimuron)

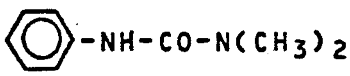

(I-D-12)
(Fenuron)

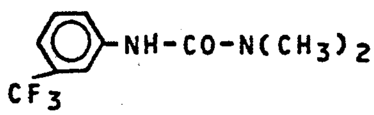

(I-D-13)
(Fluometuron)

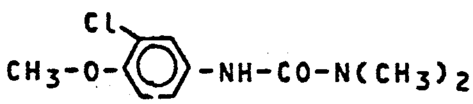

(I-D-14)
(Metoxuron)

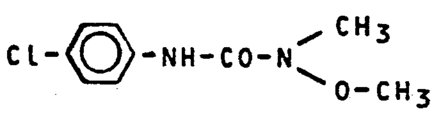

(I-D-15)
(Monolinuron)

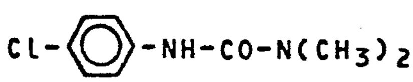

(I-D-16)
(Monuron)

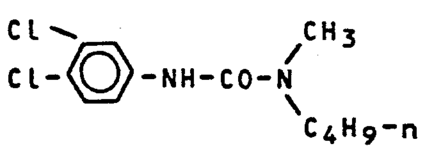

(I-D-17)
(Neburon)

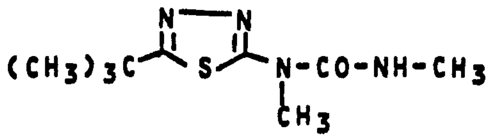

(I-D-18)
(Tebuthiuron)

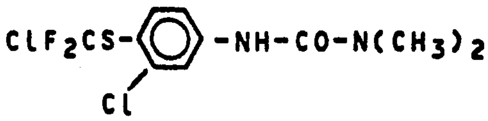

(I-D-19)
(Thiochlormethyl)

(E) Carboxanilid-Derivate der Formeln

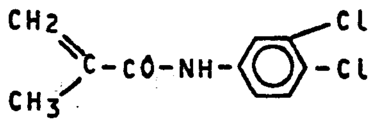

(I-E-1)
(Chlorancryl)

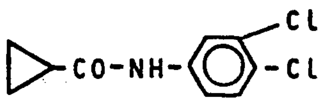

(I-E-2)
(Cypromid)

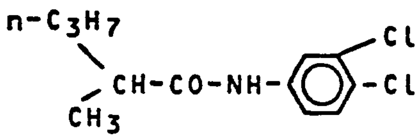

(I-E-3)
(Karsil)

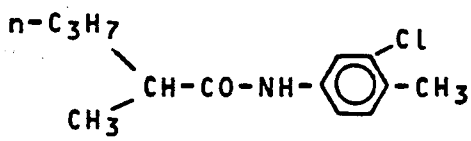

(I-E-4)
(Pentanochlor)

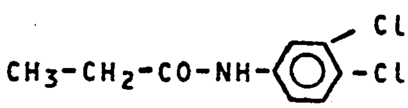

(I-E-5)
(Propanil)

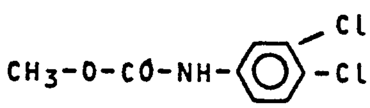

(I-E-6)
(Swep)

(F) Uracil-Derivate der Formeln

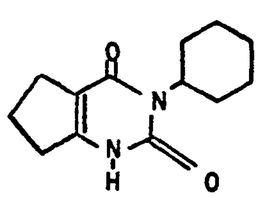

(I-F-1)
(Lenacil)

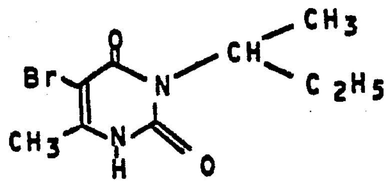

(I-F-2)
(Bromacil)

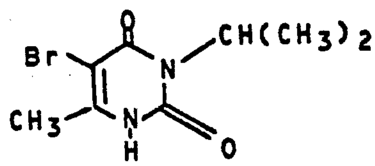

(I-F-3)
(Isocil)

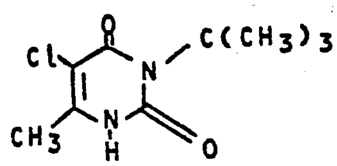

(I-F-4)
(Terbacil)

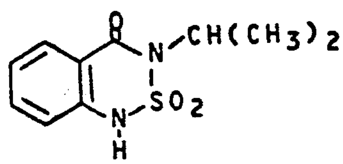

(I-F-5)
(Bentazon)

(G) Biscarbamat-Derivate der Formeln

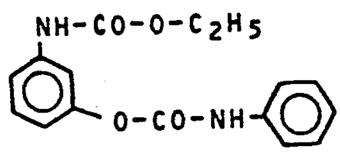

(I-G-1)
(Desmedipham)

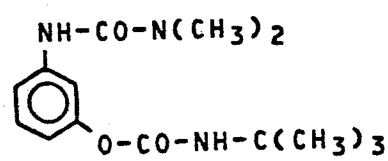

(I-G-2)
(Karbutilate)

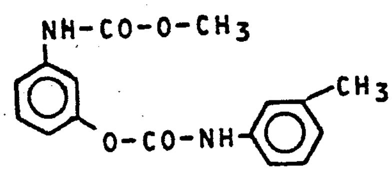

(I-G-3)
(Phenmedipham)

(H) Pyridazinon-Derivate der Formeln

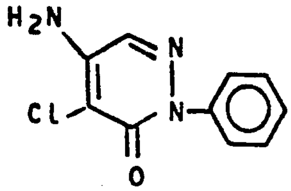

(I-H-1)
(Pyrazone)

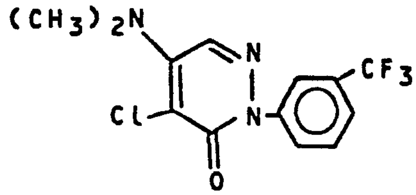

(I-H-2)
(Metflurazone)

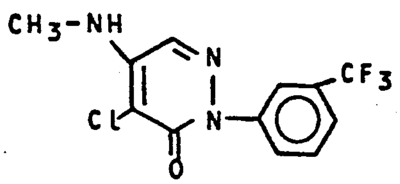

(I-H-3)
(Norflurazon)

(J) Hydroxybenzonitril-Derivate der Formeln

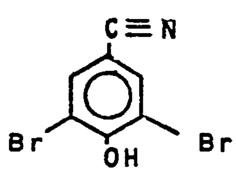

(I -J-1)
(Bromoxynil)

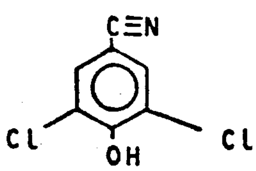

(I -J-2)
(Chloroxynil)

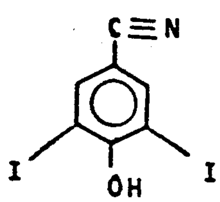

(I -J-3)
(Ioxynil)

Die Photosynthesehemmer-Wirkstoffe der Formeln (I-A)bis (I-J) sind bekannt (vergl. z. B. Carl Fedtke, Biochemistry and Physiology of Herbicide Action, Springer-Verlag, 1982).

Die weiterhin als Mischungskomponente zu verwendenden substituierten Azolyl-Derivate sind durch die Formeln (II),(III) und (IV) allgemein definiert.In dieser Formel stehen vorzugsweise.

$R^1$ für Wasserstoff oder Alkoxycarbonylmethyl mit 1 bis 4 Kohlenstoffatomen im Alkylteil;

$R^2$ für Wasserstoff oder geradkettiges bzw. verzweigtes Alkyl mit 1 bis 4 Kohlenstoffatomen;

$R^3$ für Wasserstoff, geradkettiges oder verzweigtes Alkyl mit 1 bis 4 Kohlenstoffatomen und geradkettiges oder verzweigtes Alkylthio mit 1 bis 4 Kohlenstoffatomen und

$R^4$ für Wasserstoff oder Chlor.

Besonders bevorzugt sind diejenigen Verbindungen der Formeln (II), (III) und (IV), in denen

$R^1$ für Wasserstoff, Methoxycarbonylmethyl oder Ethoxycarbonylmethyl steht;

$R^2$ für Wasserstoff oder geradkettiges bzw. verzweigtes Alkyl mit 1 bis 4 Kohlenstoffatomen steht;

$R^3$ für Wasserstoff, Methyl, Methylthio oder Ethylthio steht und

$R^4$ für Wasserstoff oder Chlor steht.

Als Beispiele für Verbindungen der Formeln (II), (III) und (IV) seien genannt:

Tabelle 1

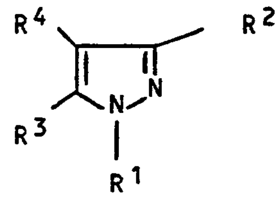

(II)

| Bsp. Nr. | $R^1$ | $R^2$ | $R^3$ | $R^4$ | Fp(°C) bzw. $^1$H-NMR-Daten (δ[ppm] in $CDCl_3$ gegen TMS) |
|---|---|---|---|---|---|
| II-1 | H | t-$C_4H_9$ | $CH_3$ | H | 162 |
| II-2 | H | n-$C_3H_7$ | $CH_3$ | H | 188 |
| II-3 | $-CH_2-COOC_2H_5$ | t-$C_4H_9$ | $CH_3$ | H | 1,27(s); 1,27(t); 2,16(s); 4,2(q); 4,75(s) |
| II-4 | $-CH_2-COOC_2H_5$ | n-$C_3H_7$ | $CH_3$ | H | 1,0(t); 1,65(m);2,4(t); 1,25(t);4,2(q),4,75(s); 2,2(s);5,9(s) |

TMS = Tetramethylsilan (als interner Standard)

s = Singulett

t = Triplett

q = Quartett

m = Multiplett

Tabelle 2

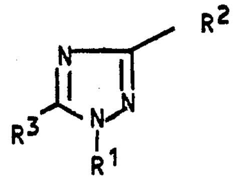

(III)

| Bsp. Nr. | $R^1$ | $R^2$ | $R^3$ | Fp(°C) bzw. Kp(°C)/mbar bzw. $^1H$-NMR-Daten (δ[ppm] in $CDCl_3$ gegen TMS) |
|---|---|---|---|---|
| III-1 | H | $CH_3$ | H | 88 |
| III-2 | H | $C_2H_5$ | H | 65 |
| III-3 | $-CH_2COOC_2H_5$ | $CH_3$ | H | 1,27(t); 4,2(q);4,83(s); 2,3(s);7,86(s) |
| III-4 | $-CH_2COOC_2H_5$ | $C_2H_5$ | H | 115/0,2 |
| III-5 | H | $n-C_3H_7$ | H | 1,0(t);1,85(m);2,9(t); 8,0(s); 13,1(s) |
| III-6 | H | $i-C_3H_7$ | H | 1,33(d); 3,1(m);7,9(s); 1,33(d) |
| III-7 | H | $n-C_3H_7$ | $-SCH_3$ | 0,97(t);1,8(m);2,7(t); 2,6(s); 12,0(s) |
| III-8 | H | $i-C_3H_7$ | $-SCH_3$ | 1,35(d);1,35(d);3,1(m); 2,6(s);11,0(s) |
| III-9 | H | H | $-SCH_3$ | 2,6(s);8,18(s);10,1(S) |
| III-10 | $-CH_2-COOC_2H_5$ | $n-C_3H_7$ | H | 160/10 |
| III-11 | $-CH_2COOC_2H_5$ | $n-C_3H_7$ | $-SCH_3$ | 145/0,2 |
| III-12 | $-CH_2COOC_2H_5$ | $i-C_3H_7$ | H | 130/1,5 |
| III-13 | $-CH_2COOC_2H_5$ | $i-C_3H_7$ | $-SCH_3$ | 1,35(d);1,35(d); 2,95(m);1,27(t);4,2(q); 4,75(s);2,6(s) |
| III-14 | $-CH_2COOC_2H_5$ | H | $-SCH_3$ | 150-60/0,1 |
| III-15 | H | $CH_3$ | $-SCH_3$ | 2,5(s);2,6(s); 10,9(s) |
| III-16 | H | $C_2H_5$ | $-SCH_3$ | 1,33(t);2,87(q);2,6(s); 9,85(s) |

| Bsp. Nr. | $R^1$ | $R^2$ | $R^3$ | Fp(°C) bzw. Kp(°C)/mbar bzw. $^1$H-NMR-Daten (δ[ppm]in $CDCl_3$ gegen TMS) |
|---|---|---|---|---|
| III-17 | H | $t-C_4H_9$ | H | 1,33(s);6,83(s); 6,83(s) |
| III-18 | H | $t-C_4H_9$ | $-SCH_3$ | 186 |
| III-19 | $-CH_2COOC_2H_5$ | $t-C_4H_9$ | H | 1,33(s);1,27(t); 4,2(w); 4,8(s); 7,6(s) |

Tabelle 3

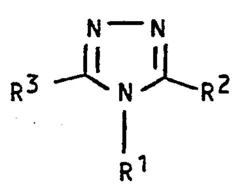

(IV)

| Bsp. Nr. | $R^1$ | $R^2$ | $R^3$ | Fp(°C) bzw. $^1$H-NMR-Daten (δ[ppm] in $CDCl_3$ gegen TMS) |
|---|---|---|---|---|
| IV-1 | H | $t-C_4H_9$ | $-SCH_3$ | 186 |
| IV-2 | $-CH_2COOC_2H_5$ | $t-C_4H_9$ | $-SCH_3$ | 1,33(s);2,6(s);1,27(t); 4,2(q);4,8(s) |

Die Verbindungen der Formeln (II), (III) und (IV) sind weitgehend bekannt (vergleiche z.B. Advances in Heterocyclic Chemistry 6 (1966); Comptes Rendus 236, 2515 (1953); Rec.Trav.Chim.Pays-Bas 86, 56-60 (1967); Angew. Chemie 94, 549; EP-OS 0 047 977) bzw.können sie nach bekannten Verfahren in analoger, einfacher Weise hergestellt werden.
So erhält man z.B. die Pyrazol-Derivate durch Umsetzung entsprechender ß-Diketone mit Hydrazin und gegebenenfalls anschließender Alkylierung am N-1.
Die Triazol-Derivate erhält man z.B. durch Umsetzung von Thiosemicarbazid mit Säurechloriden, wobei die Triazolyl-5-mercapto-Derivate entstehen. Diese können gegebenenfalls S-alkyliert bzw. oxidativ mit Salpetersäure entschwefelt werden. Zuletzt erfolgt gegebenenfalls eine Alkylierung am N-1 (vergleiche auch die Herstellungsbeispiele).

Die Gewichtsverhältnisse der Wirkstoffe in den neuen Wirkstoffkombinationen können in relativ großen Bereichen schwanken. Im allgemeinen entfallen auf ein Gewichtsteil Photosynthesehemmer-Wirkstoff (herbizider Wirkstoff) 0,25 bis 100, vorzugsweise 5 bis 50, insbesondere 10 bis 20, Gew.-Teile Azolyl-Derivat der Formeln (II), (III) bzw. (IV) (Synergist).

Die Photosynthesehemmer-Wirkstoffe weisen starke herbizide Wirkungen auf. Dennoch besitzen sie gegen einige Unkräuter, wie z. B. Galium aparine, Ipomoea hederacea, Datura stramonium, Cirsium arrense, Convolvulus arvensis oder Solanum nigrum und einige Ungräser, wie z. B. Agropyron repens, Avena fatua, Cynodon dactylon, Cyperus

ssp. und Colium rigidum eine nicht immer ausreichende Wirkung. Die erfindungsgemäßen Wirkstoffkombinationen dehnen das Wirkungsspektrum der Verbindungen der Formeln (I-A bis I-J) aus und ermöglichen dadurch eine Bekämpfung dieser durch die herbiziden Wirkstoffe alleine nur schwer oder gar nicht bekämpfbaren Unkräuter.

Die erfindungsgemäßen Wirkstoffkombinationen können z.B. bei den folgenden Pflanzen verwendet werden:

Dikotyle Unkräuter der Gattungen: Sinapis, Lepidium, Galium, Stellarium, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffkombinationen ist jedoch keineswegs auf diese Gattungen beschränkt, sondern erstreckt sich in gleicher Weise auch auf andere Pflanzen.

Die erfindungsgemäßen Wirkstoffkombinationen zeigen insbesondere neben einer guten Wirkung gegen grasartige Unkräuter auch eine gute herbizide Wirkung bei breitblättrigen Unkräutern.

Die erfindungsgemäßen Wirkstoffkombinationen können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsionskonzentrate, Wirkstoff-imprägnierte Natur- und synthetische Stoffe, Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anoranischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige,

körnige oder latexförmige Polymere verwendet werden, wie Gummiarabicum, Polyvinylalkohol, Polyvinylacetat.

In den Formulierungen können als weitere Zusätze Farbstoffe wie anorganische Pigmente, z.B. Eisenoxid, Titanoxid, Ferrocyanblau und organische Farbstoffe, wie Alizarin-, Azo-, Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoffkombination, vorzugsweise zwischen 0,5 und 90 %.

Die erfindungsgemäßen Wirkstoffkombinationen werden im allgemeinen in Form von Fertigformulierungen zur Anwendung gebracht. Die in den Wirkstoffkombinationen enthaltenen Wirkstoffe können aber auch als Einzelformulierungen bei der Anwendung gemischt, d.h. in Form von Tankmischungen zur Anwendung gebracht werden.

Die neuen Wirkstoffkombinationen können als solche oder in ihren Formulierungen weiterhin auch in Mischung mit anderen bekannten Herbiziden Verwendung finden, wobei wiederum Fertigformulierungen oder Tankmischungen möglich sind. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Die neuen Wirkstoffkombinationen können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Stäuben oder Streuen.

Die erfindungsgemäßen Wirkstoffkombinationen können sowohl vor als auch nach der Einsaat ebenso wie nach dem Auflaufen der Pflanzen gemeinsam oder in getrennten Anwendungen ausgebracht werden. Hierbei spielt die Reihenfolge der Anwendungen keine Rolle.

Beim Einsatz der erfindungsgemäßen Synergisten kann die übliche Aufwandmenge des Herbizids der Formeln (I-A bis I-J) verringert werden. Die Aufwandmenge an herbizidem Photosynthesehemmer-Wirkstoff liegt bei Flächenbehandlung zwischen 0,01 und 3,0 kg/ha, vorzugsweise zwischen 0,05 und 2,0 kg/ha.

Die Aufwandmenge an synergistischem Azolyl-Derivat (II), (III) bzw. (IV) liegt bei Flächenbehandlung zwischen 0,1 und 10 kg/ha, vorzugsweise zwischen 0,5 und 3 kg/ha.

Die gute herbizide Wirkung der erfindungsgemäßen Wirkstoffkombinationen geht aus den nachfolgenden Beispielen hervor. Während die einzelnen Wirkstoffe in der herbiziden Wirkung Schwächen aufweisen, zeigen die Kombinationen eine Unkrautwirkung, die über eine einfache Wirkungssummierung hinausgeht.

Ein synergistischer Effekt liegt bei Herbiziden immer dann vor, wenn die herbizide Wirkung der Wirkstoffkombination größer ist als die Summe der Wirkungen der applizierten Wirkstoffe.

Beispiel A

Pre-emergence-Test

Lösungsmittel: 5 Gew.-Teile Aceton
Emulgator: 1 Gew.-Teil Alkylarylpolyglykolether

Zur Herstellung einer zweckmäßigen Wirkstoffzubereitung vermischt man 1 Gew.-Teil herbiziden Wirkstoffs bzw. Synergisten bzw. eines Gemisches aus herbizidem Wirkstoff und Synergisten mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit einer Herbizid-Zubereitung bzw. mit der Synergisten-Zubereitung bzw. mit der Zubereitung aus Synergisten und herbizidem Wirkstoff begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffes pro Flächeneinheit. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100 % = totale Vernichtung

Wirkstoffe, Aufwandmenge und Resultate gehen aus den nachfolgenden Tabellen hervor.

Pre-emergence-Test

Tabelle $A_1$:

Synergistische Wirkung von Azolyl-Derviaten (II, III, IV) (= Synergist S) und 4-Amino-6-tert.-butyl-3-methylthio-1,2,4-triazin-5-on (I-A-1) (= Herbizid H) an Ipomoea hederacea. Die Aufwandmenge in kg/ha bezieht sich auf den Gehalt an Wirkstoff.

| Struktur des Synergisten | (S) kg/ha | (H) kg/ha | % Schäden bei Ipomoea hederacea H | S | H+S |
|---|---|---|---|---|---|
| 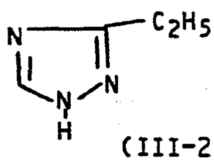 (III-2) | 0,5 | 0,05 | 0 | 0 | 20 |
| | 2 | 0,05 | 0 | 0 | 50 |
| | 0,5 | 0,15 | 30 | 0 | 80 |
| | 2 | 0,15 | 30 | 0 | 90 |
| 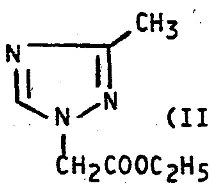 (III-3) | 0,5 | 0,05 | 0 | 0 | 0 |
| | 2 | 0,05 | 0 | 0 | 0 |
| | 0,5 | 0,15 | 10 | 0 | 20 |
| | 2 | 0,15 | 10 | 0 | 20 |
| 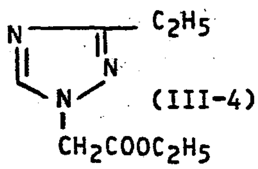 (III-4) | 0,5 | 0,05 | 0 | 0 | 0 |
| | 2 | 0,05 | 0 | 0 | 0 |
| | 0,5 | 0,15 | 10 | 0 | 20 |
| | 2 | 0,15 | 10 | 0 | 20 |
| 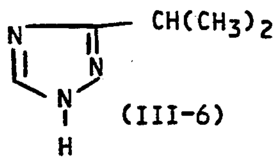 (III-6) | 0,5 | 0,05 | 0 | 0 | 20 |
| | 2 | 0,05 | 0 | 0 | 20 |
| | 0,5 | 0,15 | 20 | 0 | 80 |
| | 2 | 0,15 | 20 | 0 | 90 |
| 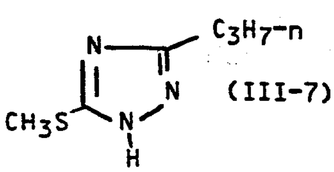 (III-7) | 0,5 | 0,05 | 10 | 0 | 10 |
| | 2 | 0,05 | 10 | 10 | 20 |
| | 0,5 | 0,15 | 20 | 0 | 50 |
| | 2 | 0,15 | 20 | 10 | 90 |

T a b e l l e $A_1$: Pre-emergence-Test / Forsetzung

| Struktur des Synergisten | (S) kg/ha | (H) kg/ha | % Schäden bei Ipomoea hederacea H | S | H+S |
|---|---|---|---|---|---|
| 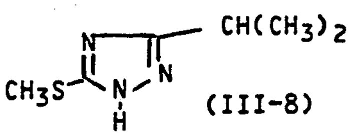 (III-8) | 0,5 | 0,05 | 10 | 0 | 0 |
| | 2 | 0,05 | 10 | 10 | 10 |
| | 0,5 | 0,15 | 20 | 0 | 30 |
| | 2 | 0,15 | 20 | 20 | 30 |
| 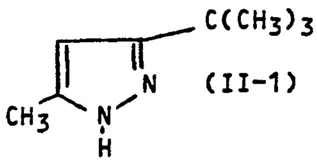 (II-1) | 0,5 | 0,05 | 10 | 0 | 80 |
| | 2 | 0,05 | 10 | 0 | 90 |
| | 0,5 | 0,15 | 20 | 0 | 100 |
| | 2 | 0,15 | 20 | 0 | 100 |
| 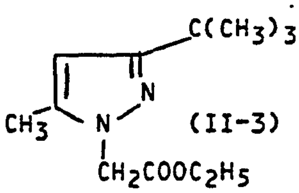 (II-3) | 0,5 | 0,05 | 0 | 0 | 20 |
| | 2 | 0,05 | 0 | 0 | 30 |
| | 0,5 | 0,15 | 10 | 0 | 60 |
| | 2 | 0,15 | 10 | 0 | 90 |
| 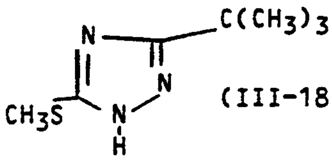 (III-18) | 0,5 | 0,05 | 10 | 0 | 0 |
| | 2 | 0,05 | 10 | 0 | 50 |
| | 0,5 | 0,15 | 30 | 0 | 60 |
| | 2 | 0,15 | 30 | 0 | 90 |
| 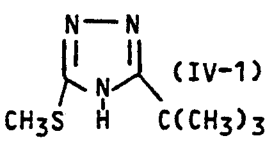 (IV-1) | 0,5 | 0,05 | 10 | 0 | 0 |
| | 2 | 0,05 | 10 | 0 | 30 |
| | 0,5 | 0,15 | 30 | 0 | 40 |
| | 2 | 0,15 | 30 | 0 | 90 |
| 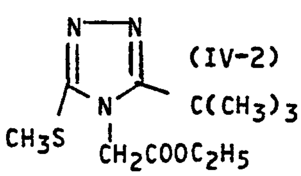 (IV-2) | 0,5 | 0,05 | 10 | 0 | 10 |
| | 2 | 0,05 | 10 | 0 | 10 |
| | 0,5 | 0,15 | 20 | 0 | 60 |
| | 2 | 0,15 | 20 | 0 | 60 |
| 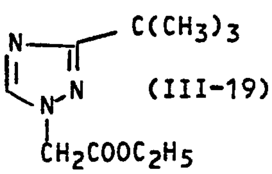 (III-19) | 0,5 | 0,05 | 0 | 0 | 10 |
| | 2 | 0,05 | 0 | 0 | 20 |
| | 0,5 | 0,15 | 10 | 0 | 50 |
| | 2 | 0,15 | 10 | 0 | 40 |

Tabelle $A_1$: Pre-emergence-Test / Fortsetzung

| Struktur des Synergisten | (S) Kg/ha | (H) Kg/ha | % Schäden bei Ipomoea hederacea H | S | H+S |
|---|---|---|---|---|---|
| 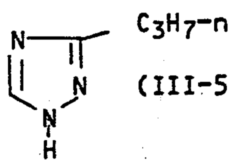 (III-5) | 0,5 | 0,05 | 30 | 10 | 100 |
| | 2 | 0,05 | 30 | 10 | 50 |
| | 0,5 | 0,15 | 30 | 10 | 90 |
| | 2 | 0,15 | 30 | 10 | 100 |
| 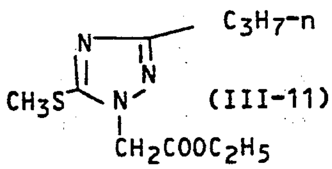 (III-11) | 0,5 | 0,05 | 20 | 0 | 20 |
| | 2 | 0,05 | 20 | 10 | 30 |
| | 0,5 | 0,15 | 50 | 0 | 30 |
| | 2 | 0,15 | 50 | 10 | 90 |
| 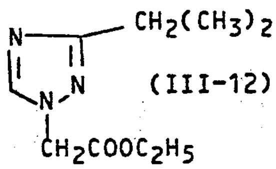 (III-12) | 0,5 | 0,05 | 20 | 0 | 20 |
| | 2 | 0,05 | 20 | 0 | 20 |
| | 0,5 | 0,15 | 50 | 0 | 90 |
| | 2 | 0,15 | 50 | 0 | 80 |
| 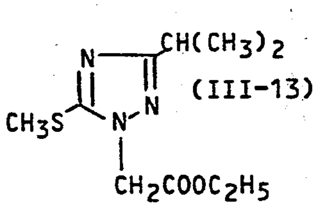 (III-13) | 0,5 | 0,05 | 20 | 10 | 10 |
| | 2 | 0,05 | 20 | 0 | 20 |
| | 0,5 | 0,15 | 50 | 10 | 40 |
| | 2 | 0,15 | 50 | 0 | 80 |
| 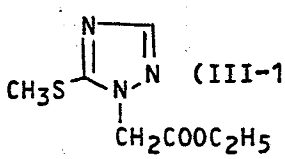 (III-14) | 0,5 | 0,05 | 20 | 0 | 30 |
| | 2 | 0,05 | 20 | 0 | 50 |
| | 0,5 | 0,15 | 50 | 0 | 50 |
| | 2 | 0,15 | 50 | 0 | 70 |
| 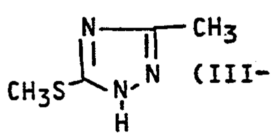 (III-15) | 0,5 | 0,05 | 20 | 0 | 20 |
| | 2 | 0,05 | 20 | 0 | 30 |
| | 0,5 | 0,15 | 50 | 0 | 40 |
| | 2 | 0,15 | 50 | 0 | 50 |

Tabelle $A_1$: Pre-emergence-Test / Fortsetzung

| Struktur des Synergisten | (S) kg/ha | (H) kg/ha | % Schäden bei Ipomoea hederacea H | S | H + S |
|---|---|---|---|---|---|
| 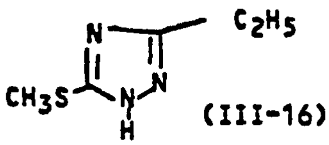 (III-16) | 0,5 | 0,05 | 20 | 0 | 20 |
| | 2 | 0,05 | 20 | 10 | 80 |
| | 0,5 | 0,15 | 50 | 0 | 50 |
| | 2 | 0,15 | 50 | 10 | 90 |
| 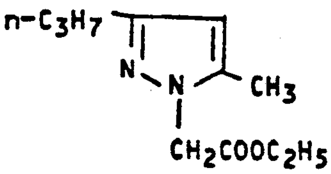 (II-4) | 0,5 | 0,05 | 20 | 0 | 20 |
| | 2 | 0,05 | 20 | 0 | 100 |
| | 0,5 | 0,15 | 50 | 0 | 50 |
| | 2 | 0,15 | 50 | 0 | 100 |
| 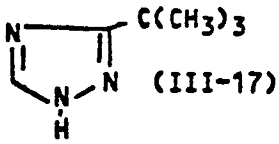 (III-17) | 0,03 | 0,1 | 30 | - | 50 |
| | 0,1 | 0,1 | 30 | - | 50 |
| | 0,3 | 0,1 | 30 | - | 50 |
| | 1 | 0,1 | 30 | - | 50 |
| | 2 | 0,1 | 30 | 30 | 50 |

Die alleinige Anwendung des Herbizids (I-A-1) in einer Konzentration von 0,5 bzw. 2,0 kg/ha führt bei Ipomoea hederacea zu einer Schädigung von 80 bzw. 95 %.

Tabelle $A_2$

Synergistische Wirkung des Azolyl-Derivats gemäß Herstellungsbeispiel (II-1) und verschiedener Photosynthesehemmer-Herbizide an Ipomoea hederacea. Die Aufwandmenge in kg/ha bezieht sich auf den Gehalt an Wirkstoff.

| Synergist | Herst.Bsp. (II-1) | 0 kg/ha | 0,5kg/ha | 2,0 kg/ha |
|---|---|---|---|---|
| Herbizide | kg/ha | % Wirkung bei Ipomoea hederacea | | |
| Metribuzin (I-A-1) | 0,05 | 30 | 90 | 90 |
| | 0,07 | 30 | 100 | 100 |
| | 0,1 | 40 | 90 | 100 |
| (I-A-2) | 0,3 | 0 | 30 | 50 |
| | 1,0 | 20 | 60 | 90 |
| Methabenzthiazuron (I-D-2) | 1,0 | 0 | 0 | 100 |
| | 3,0 | 40 | 50 | 100 |
| Linuron (I-D-1) | 1,0 | 60 | 50 | 90 |
| - | 0 | 0 | 0 | 0 |

Herstellungsbeispiele

Beispiel (II-1)

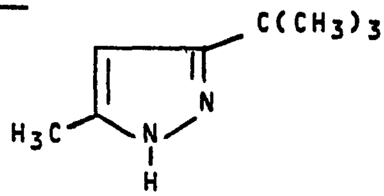

Zu 28,4g (0,49 Mol) 85%-igem Hydrazinhydrat in 600 ml Wasser werden bei 10°C 69,5g (0,49 Mol) 5,5 -Dimethylhexandion-(2,4) (J.Chem.Soc. 121, 937) im Verlauf von 4 Stunden zugetropft. Man läßt das Reaktionsgemisch 12 Stunden bei Raumtemperatur nachrühren und kühlt anschließend auf 5 -10°C ab. Der ausgefallene Niederschlag wird abgesaugt, mit kaltem Wasser gewaschen und getrocknet. Man erhält 49g (72 % der Theorie) 3-tert.-Butyl-5-methyl-pyrazol vom Schmelzpunkt 162°C.

Beispiel (II-3)

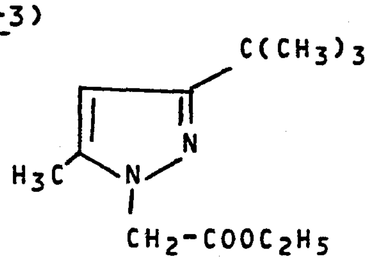

Eine Lösung von 2,16 g (0,094 Mol) Natrium in 50 ml Methanol wird mit 13g (0,094 Mol) 3-tert.-Butyl-5-methyl-pyrazol (Bsp.II-1) versetzt und 2 Stunden unter Rückfluß erhitzt. Nach dem Abkühlen wird zur Trockne eingeengt; Reste von Methanol werden durch zweifaches Abdestillieren mit Toluol entfernt. Der Rückstand wird in 150 ml Acetonitril aufgenommen und tropfenweise mit 15,7ml (0,094 Mol) Bromessigsäureethylester versetzt, wobei sich die Reaktionsmischung erwärmt. 2 Stunden Nachrühren unter

Rückfluß und anschließend 12 Stunden bei Raumtemperatur vervollständigt die Umsetzung. Nach dem Einengen wird das Reaktionsgemisch in Essigester aufgenommen, das ausgefallene Natriumbromid wird abgesaugt und das Filtrat erneut eingeengt. Man erhält 22,1g (98 % der Theorie) 1-Ethoxycarbonylmethyl-3-tert.-butyl-5-methyl-pyrazol.

Beispiel (III-2)

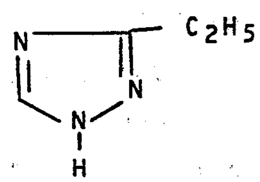

Eine Lösung von 100 ml konzentrierter Salpetersäure und 1g Natriumnitrit in 200 ml Wasser wird bei 30 - 40°C portionsweise mit 100 g 3-Ethyl-5-mercapto-1,2,4-triazol versetzt. Nach Beendigung der Reaktion wird die Lösung mit gesättigter Natriumcarbonatlösung bei 0 - 10°C neutralisiert. Die dabei entstehende Suspension wird zur Trockne eingeengt, der Rückstand mit 500 ml Ethanol aufgenommen, abgesaugt und das Filtrat eingeengt. Der Rückstand wird aus Toluol umkristallisiert. Man erhält 53 g (70 % der Theorie) 3-Ethyl-1,2,4-triazol vom Schmelzpunkt 65°C.

Beispiel (III-4)

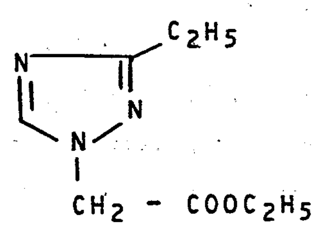

Entsprechend der Umsetzung gemäß Beispiel (II-3) erhält man bei einem einmolaren Ansatz 1-Ethoxycarbonylmethyl-3-ethyl-1,2,4-triazol in 54%-iger Ausbeute.

Beispiel (III-16)

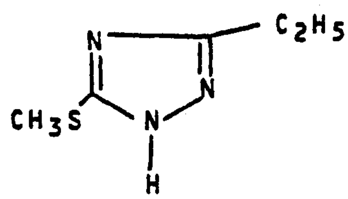

Zu einer Lösung von 130g (1 Mol) 3-Ethyl-5-mercapto-1,2,4-triazol in 500 ml Methanol werden 56,1g (1 Mol) gepulvertes Kaliumhydroxid gegeben. Zu dieser Mischung werden 62,27 ml (1 Mol) Methyliodid getropft. Anschließend läßt man die Reaktionslösung je zwei Stunden bei Raumtemperatur und unter Rückfluß nachrühren. Nach dem Abkühlen wird eingeengt, der Rückstand in 200 ml Methylenchlorid aufgenommen, mit Wasser gewaschen, über Natriumsulfat getrocknet und eingeengt. Man erhält 125g (87 % der Theorie) 3-Ethyl-5-methylthio-1,2,4-triazol.

In analoger Weise zu diesen Herstellungsbeispielen und in Anlehnung an literaturbekannte Verfahren erhält man die Verbindungen der allgemeinen Formeln (II), (III) und (IV) der obigen Tabellen 1, 2 und 3.

Patentansprüche

1. Herbizide Mittel, gekennzeichnet durch einen Gehalt an einer Wirkstoffkombination, bestehend aus

(a) einem Photosynthesehemmer-Wirkstoff (Herbizid) und

(b) einem substituierten Azolyl-Derivat der allgemeinen Formeln (II), (III) und (IV) (Synergisten)

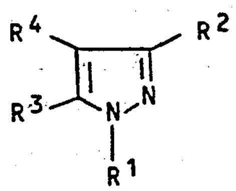

(II)

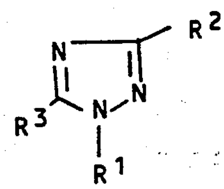

(III)

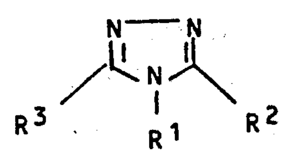

(IV)

in welchen

$R^1$ für Wasserstoff oder Alkylcarbonylmethyl steht,

$R^2$ für Wasserstoff oder Alkyl steht,

$R^3$ für Wasserstoff, Alkyl oder Alkylthio steht und

$R^4$ für Wasserstoff oder Halogen steht.

2. Herbizide Mittel gemäß Anspruch 1, dadurch gekennzeichnet, daß sie als Photosynthesehemmer-Wirkstoffe (Herbizide) Verbindungen aus den nachfolgend genannten Wirkstoffgruppen der Formeln (I-A) bis (I-J) enthalten:

(A) Triazinon-Derivate der Formel

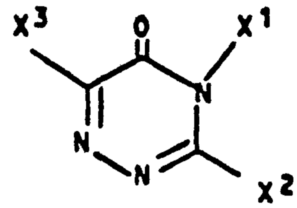

(I-A)

in welcher

$X^1$ für Amino, gegebenenfalls substituiertes Alkylidenamino oder Alkyl mit 1 bis 2 Kohlenstoffatomen steht;

$X^2$ für Alkylthio mit 1 bis 2 Kohlenstoffatomen, Alkyl- und Dialkylamino mit jeweils 1 bis 2 Kohlenstoffatomen in jedem Alkylteil oder Alkyl mit 1 bis 4 Kohlenstoffatomen steht; und

$X^3$ für gegebenenfalls durch Halogen substituiertes tert.-Butyl oder gegebenenfalls substituiertes Phenyl steht;

(B) Triazindion-Derivate der Formel

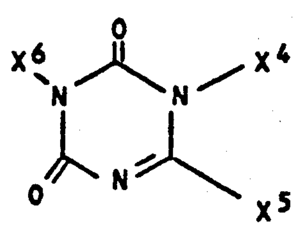

(I-B)

in welcher

$X^4$ für Amino, gegebenenfalls substituiertes Alkylidenamino oder Alkyl mit 1 bis 2 Kohlenstoffatomen steht;

$X^5$ für Alkylthio mit 1 bis 2 Kohlenstoffatomen, Alkyl- und Dialkylamino mit jeweils 1 bis 2 Kohlenstoffatomen in jedem Alkylteil oder Alkyl mit 1 bis 4 Kohlenstoffatomen steht; und

$X^6$ für Alkyl mit 1 bis 6 Kohlenstoffatomen oder gegebenenfalls substituiertes Phenyl steht;

(C) Triazin-Derivate der Formel

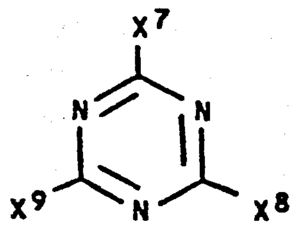

(I-C)

in welcher

$X^7$ für Chlor, Alkoxy oder Alkylthio mit jeweils 1 bis 2 Kohlenstoffatomen steht;

$X^8$ für Alkylamino mit 1 bis 4 Kohlenstoffatomen im Alkylteil steht; und

$X^9$ für gegebenenfalls durch Cyano substituiertes Alkyl mit 1 bis 4 Kohlenstoffatomen steht;

(D) Harnstoff-Derivate der Formel

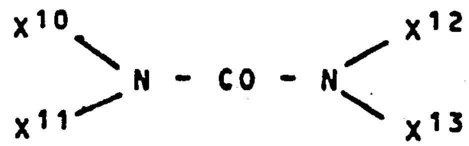

(I-D)

in welcher

$X^{10}$ für gegebenenfalls substituiertes Phenyl, Benz-thiazolyl oder gegebenenfalls substituiertes Thiadiazolyl steht;

$X^{11}$ für Wasserstoff oder Methyl steht;

$X^{12}$ für Methyl steht; und

$X^{13}$ für Wasserstoff, Alkyl mit 1 bis 4 Kohlenstoffatomen, Alkoxy mit 1 bis 2 Kohlenstoffatomen oder Alkinyl mit 2 bis 4 Kohlenstoffatomen steht;

(E) Carboxanilid-Derivate der Formel

$$X^{14} - CO - NH - X^{15} \qquad (I\text{-}E)$$

in welcher

$X^{14}$ für Alkyl mit bis zu 6 Kohlenstoffatomen, Alkoxy mit 1 bis 4 Kohlenstoffatomen, Alkenyl mit 2 bis 4 Kohlenstoffatomen oder Cycloalkyl mit 3 bis 6 Kohlenstoffatomen steht; und

$X^{15}$ für gegebenenfalls substituiertes Phenyl steht;

(F) Uracil-Derivate der Formel

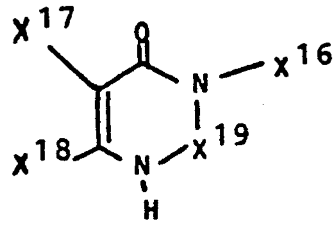

(I-F)

in welcher

$X^{16}$ für Alkyl mit 1 bis 6 Kohlenstoffatomen oder Cycloalkyl mit 5 bis 7 Kohlenstoffatomen steht;

$X^{17}$ für Halogen steht;

$X^{18}$ für Alkyl mit 1 bis 2 Kohlenstoffatomen steht, oder

$X^{17}$ und $X^{18}$ gemeinsam für eine gegebenenfalls substituierte Alkylenkette oder einen gegebenenfalls substituierten anellierten Benzolring stehen; und

$X^{19}$ für die -CO- oder $-SO_2$-Gruppe steht;

(G) Biscarbamat-Derivate der Formel

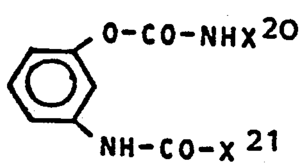

(I-G)

in welcher

$X^{20}$ für Alkyl mit 1 bis 4 Kohlenstoffatomen oder gegebenenfalls substituiertes Phenyl steht; und

$X^{21}$ für Alkoxy mit 1 bis 4 Kohlenstoffatomen oder Dialkylamino mit 1 bis 2 Kohlenstoffatomen in jedem Alkylteil steht;

(H) Pyridazinon-Derivate der Formel

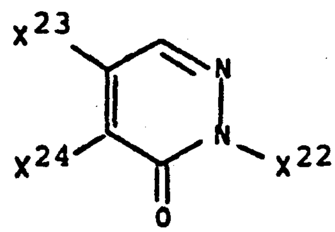

(I-H)

in welcher

$X^{22}$ für gegebenenfalls substituiertes Phenyl steht;

$X^{23}$ für Amino, Alkylamino oder Dialkylamino mit jeweils 1 oder 2 Kohlenstoffatomen in jedem Alkylteil steht; und

$X^{24}$ für Halogen steht;

(J) Hydroxybenzonitril-Derivate der Formel

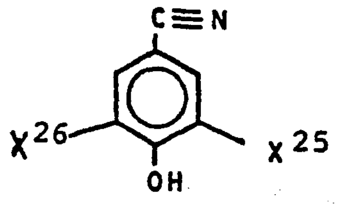

(I-J)

in welcher

$X^{25}$ für Halogen steht; und

$X^{26}$ für Halogen steht.

3. Herbizide Mittel gemäß Anspruch 1, dadurch gekennzeichnet, daß sie als Photosynthesehemmer-Wirkstoff (Herbizid) 4-Amino-6-tert.-butyl-3-methylthio-1,2,4-triazin-5-on der Formel (I-A-1) (Metribuzin)

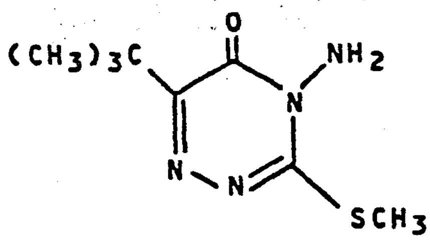

enthalten.

4. Herbizide Mittel gemäß Anspruch 1, dadurch gekennzeichnet, daß in der Wirkstoffkombination das Gewichtsverhältnis von (1) Photosynthesehemmer-Wirkstoff (Herbizid) zu (2) dem substituiertem Azolyl-Derivat der Formeln (II), (III) bzw. (IV) (Synergist) zwischen 1:0,25 und 1:100 liegt.

5. Herbizide Mittel gemäß Anspruch 4, dadurch gekennzeichnet, daß das angegebene Gewichtsverhältnis zwischen 1 : 5 und 1 : 50 liegt.

6. Herbizide Mittel gemäß Anspruch 5, dadurch gekennzeichnet, daß das angegebene Gewichtsverhältnis zwischen 1 : 10 und 1 : 20 liegt.

7. Verwendung von Wirkstoffkombinationen gemäß Ansprüchen 1 bis 6 zur Bekämpfung von Unkraut.

8. Verfahren zur Herstellung von herbiziden Mitteln, dadurch gekennzeichnet, daß man eine Wirkstoffkombination gemäß Ansprüchen 1 bis 6 mit Streckmitteln und/oder oberflächenaktiven Mitteln vermischt.

9. Verwendung von substituierten Azolyl-Derivaten der Formeln (II), (III) und (IV) gemäß Anspruch 1 als Synergisten in Kombination mit Photosynthesehemmer-Herbiziden.

10. Verwendung von substituierten Azolyl-Derivaten der Formeln (II), (III) und (IV) gemäß Anspruch 1 als Synergisten in Kombination mit dem herbiziden Wirkstoff 4-Amino-6-tert.-butyl-3-methylthio-1,2,4-triazin-5-on (Metribuzin) der Formel (I-A-1) gemäß Anspruch 3.